# EUROPEAN PATENT SPECIFICATION

(11) **EP 1 848 462 B1**
(45) Date of publication and mention of the grant of the patent: **26.10.2011**
(21) Application number: 06723047.4
(22) Date of filing: 16.02.2006
(51) Int. Cl.: A61K 47/48, A61K 39/395

(54) **METHODS FOR TREATING CANCER USING AN IMMUNOTOXIN COMPRISING AN EXOTOXIN A MOIETY HAVING A FURIN CLEAVAGE SITE REPLACED WITH A CANCER-ASSOCIATED PROTEASE SITE CLEAVED BY MMP-2 OR MMP-9**
VERFAHREN ZUR BEHANDLUNG VON KREBS UNTER VERWENDUNG EINES IMMUNOTOXINS MIT EINER EXOTOXIN-A-GRUPPIERUNG, BEI DER EINE FURINSCHNITTSTELLE DURCH EINE VON MMP-2 ODER MMP-9 GESPALTENE KREBSASSOZIIERTE PROTEASESTELLE ERSETZT IST
TRAITEMENT DU CANCER À L'AIDE D'UNE IMMUNOTOXINE COMPRENANT UNE EXOTOXINE A DONT LE SITE DE CLIVAGE PAR LA FURINE EST REMPLACÉ PAR UN SITE DE PROTÉASE ASSOCIÉE AU CANCER QUI EST CLIVÉ PAR MMP-2 OU MMP-9

(30) Priority: 16.02.2005 US 653112 P
(43) Date of publication of application: 31.10.2007
(73) Proprietor: University of Zürich, 8006 Zürich (CH)
(72) Inventor: ZANGEMEISTER-WITTKE, Uwe, CH-8424 Embrach (CH); DI PAOLO, Claudio, CH-8041 Zürich (CH)
(74) Representative: Vossius & Partner
(86) International application number: PCT/EP2006/001421
(87) International publication number: WO 2006/087196

(56) References cited:
- WO-A-98/20135
- WO-A-2004/096271
- LIU S ET AL: "Tumor cell-selective cytotoxicity of matrix metalloproteinase-activa ted anthrax toxin." CANCER RESEARCH. 1 NOV 2000, vol. 60, no. 21, 1 November 2000 (2000-11-01), pages 6061-6067, XP002311242 ISSN: 0008-5472
- FRANKEL ARTHUR E ET AL: "Peptide toxins directed at the matrix dissolution systems of cancer cells." PROTEIN AND PEPTIDE LETTERS. FEB 2002, vol. 9, no. 1, February 2002 (2002-02), pages 1-14, XP002408612 ISSN: 0929-8665
- CHIRON M F ET AL: "Pseudomonas exotoxin exhibits increased sensitivity to furin when sequences at the cleavage site are mutated to resemble the arginine-rich loop of diphtheria toxin" MOLECULAR MICROBIOLOGY, BLACKWELL SCIENTIFIC, OXFORD, GB, vol. 22, no. 4, November 1996 (1996-11), pages 769-778, XP002391666 ISSN: 0950-382X
- LIU S ET AL: "Targeting of tumor cells by cell surface urokinase plasminogen activator-dependent anthrax toxin" JOURNAL OF BIOLOGICAL CHEMISTRY, AMERICAN SOCIETY OF BIOLOCHEMICAL BIOLOGISTS, BIRMINGHAM,, US, vol. 276, no. 21, 25 May 2001 (2001-05-25), pages 17976-17984, XP002974279 ISSN: 0021-9258
- RAJAGOPAL V ET AL: "Recombinant toxins that bind to the urokinase receptor are cytotoxic without requiring binding to the alpha2-macroglobulin receptor" JOURNAL OF BIOLOGICAL CHEMISTRY, AMERICAN SOCIETY OF BIOLOCHEMICAL BIOLOGISTS, BIRMINGHAM,, US, vol. 275, no. 11, 17 March 2000 (2000-03-17), pages 7566-7573, XP002979023 ISSN: 0021-9258
- DEBINSKI W ET AL: "MONOVALENT IMMUNOTOXIN CONTAINING TRUNCATED FORM OF PSEUDOMONAS EXOTOXIN AS POTENT ANTITUMOR AGENT" CANCER RESEARCH, AMERICAN ASSOCIATION FOR CANCER RESEARCH, BALTIMORE, MD, US, vol. 52, no. 19, 1 October 1992 (1992-10-01), pages 5379-5385, XP001246647 ISSN: 0008-5472
- CHEN EMILY I ET AL: "A unique substrate recognition profile for matrix metalloproteinase-2", THE JOURNAL OF BIOLOGICAL CHEMISTRY, vol. 277, no. 6, 2002,

## Description

The invention relates to immunotoxins containing modified toxins useful as therapeutics against cancer. Specifically, the internal furin site in the protein *Pseudomonas* exotoxin A is replaced by a cleavage site that is cleaved by proteases associated with tumour cells.

Immunotherapy has emerged as a potentially effective approach to combat cancer. Murine and humanized/chimeric antibodies, and their respective antibody fragments, directed against tumor-associated antigens ("TAAs") have been used for diagnosis and therapy of certain human cancers.²⁻¹⁰ Unconjugated, toxin-conjugated, and radiolabeled forms of these antibodies have been used in such therapies.

Exotoxin A (ETA) is one of the toxic proteins released by pathogenic strains of *Pseudomonas aeruginosa¹⁶.* It is secreted as a proenzyme with a molecular weight of 66,000 daltons¹⁷. Exotoxin A is translocated into susceptible mammalian cells, where covalent alteration of the molecule renders it enzymatically active. *Pseudomonas* exotoxin A irreversibly blocks protein synthesis in cells by adenosine diphosphate-ribosylating a post-translationally modified histidine residue of elongation factor-2, called diphthamide, and induces apoptosis.¹ A truncated version of ETA, containing the domains for inducing cell death, but lacking the cell-binding domain, prevents the ETA portion from entering cells absent targeting by the antibody portion of the immunotoxin.

One specific approach has been targeted therapy using the VB4-845 immunotoxin. VB4-845 is an immunotoxin comprised of a single-chain Fv recombinant antibody fragment that is fused to a truncated form of *Pseudomonas* exotoxin A (ETA 252-608) (WO 2004/096271). The antibody fragment referred to as 4D5MOCB was obtained through the humanization and stabilization of the MOC31 mouse monoclonal antibody, which specifically binds to Ep-CAM.¹³⁻¹⁵

Ep-CAM (for Epithelial Cell Adhesion Molecule, which is also known as 17-1A, KSA, EGP-2 and GA733-2) is a transmembrane protein that is highly expressed in many solid tumors, including carcinomas of the lung, breast, ovary, colorectum, and squamous cell carcinoma of the head and neck, but weakly expressed in most normal epithelial tissues. The role of Ep-CAM in cancer formation remains unclear; however, its expression correlates with the rate of cellular proliferation. Ep-CAM-specific antibodies have been used to image and detect primary tumors and metastases in patients with small cell lung cancer and non-small cell lung cancer. Among anti-Ep-CAM MAbs, PANOREX®, which is a murine monoclonal antibody also known as edrecolomab, had been approved for the treatment of colon cancer in Germany, and is in clinical trials in the United States.¹¹⁻¹² Of note, however, PANOREX^{®} treatment has been associated with undesirable side effects, including abdominal cramps, nausea, transient diarrhea and cutaneous urticarial lesions.²¹⁻²⁴ Clinical trials with other Ep-CAM-targeted antibodies have been less successful; antibody BIS-1 was associated with peripheral vasoconstriction, dyspnea and fever, and antibody 3622W94 was associated with acute necrotizing pancreatitis.¹⁸⁻²⁰ The search for an effective, low-toxicity, anti-Ep-CAM antibody continues: a fully humanized anti-Ep-CAM antibody, MT201, purported to act via Antibody-Dependent Cellular Cytotoxicity ("ADCC"), has been reported.²⁵ A humanized, stabilized, single-chain, anti-Ep-CAM antibody, 4D5MOC-B, which is derived from murine monoclonal antibody MOC31, has also been developed, and is described in International Patent Application No. PCT/EP00/03176, Publication No. WO 00/61635, filed April 10, 2000 and published October 19, 2000, and in Willuda et al.²⁶

In the ETA moiety of VB4-845, an internal furin site is exposed once the molecule unfurls inside the endosome due to a lower pH. Furin is then able to cleave the immunotoxin at the furin-sensitive site allowing efficient trafficking of the toxin and ultimately resulting in cell death. Furin is over-expressed in a variety of tumors but is also well expressed in a wide range of normal tissue, and, thus, does not provide added tumour specificity for the immunotoxin resulting in toxicity to some normal tissues. It would be advantageous to replace the non-tumour specific furin-sensitive site with one that increases the tumour specificity of the cancer-targeting immunotoxin.

Matrix Metallo-Proteases (MMPs) are trans-membrane proteases that are anchored in the plasma membrane with their catalytic site exposed on the external surface. MMPs constitute a family of over 21 proteolytic members, and participate in the degradation of a wide spectrum of extra cellular and non-matrix proteins. These proteases are widely expressed and have pivotal roles in many normal and pathological processes, however in normal cells their expression is tightly regulated and more limited than furin. Their dysregulated or over-expression is associated with the diseased state such as inflammation, autoimmune diseases, cardiovascular disease and cancer. Increasing levels of expression have been associated with well-differentiated and more invasive tumor cell lines. In addition, certain MMPs have shown a more enhanced cytoplasmic expression in highly invasive and metastatic tumors, and changes in localization and intracellular distribution of MMP-2 is associated with the transition from benign prostate epithelium to high grade prostatic intraepithelial neoplasia (Upadhyay J, Shekarriz B, Nemeth JA, Dong Z, Cummings GD, Fridman R, Sakr W, Grignon DJ, Cher ML. Membrane type 1-matrix metalloproteinase (MT1-MMP) and MMP-2 immunolocalization in human prostate:
change in cellular localization associated with high-grade prostatic intraepithelial neoplasia. Clin Cancer Res. 1999 Dec;5(12):4105-10). Moreover, increased MMP-2 expression by malignant prostatic epithelia is an independent predictor of decreased
prostate cancer disease-free survival (Trudel D, Fradet Y, Meyer F, Harel F, Tetu B.
Significance of MMP-2 expression in prostate cancer: an immunohistochemical study
Cancer Res. 2003 Dec 1;63(23):8511-5).

Modified anthrax toxins have been made wherein the furin site is replaced with a metalloproteinase site (Liu et al., Cancer Research, 2000, Vol. 60, No. 21, pp. 6061-6067, and Frankel et al., Protein and Peptide Letters, 2002, Vol. 9, No. 1, p.1-4).

The invention relates to a modified toxin comprising
(a) an antibody or antibody fragment that binds to Ep-CAM on the surface of a cancer cell; and
(b) a modified toxin comprising a Pseudomonas exotoxin A (ETA) moiety having a furin site replaced with a cancer-associated protease site recognized by metalloproteinase-2 (MMP-2), metalloproteinase-9 (MMP-9) or a combination thereof.

The immunotoxins of the present invention may be used to treat various forms of cancer such as colorectal cancer, breast cancer, ovarian cancer, pancreatic cancer, head and neck cancer, bladder cancer, gastrointestinal cancer, prostate cancer, small cell and non small cell lung cancer, sarcomas, gliomas, T- and B-cell lymphomas.

In one embodiment of the invention the immunotoxin is internalized by the cancer cell. In a particular embodiment, the present invention provides an immunotoxin comprising a mutated VB4-845 immunotoxin having the furin site of the ETA moiety, RQPR, replaced with a cancer-associated protease site.

In an embodiment of the invention, the cancer-associated protease site comprises the sequence GPLGMLSQ specifically recognized by MMP-2 and MMP-9. In another embodiment, the cancer-associated protease comprises the sequence GPLGLWAQ which is also recognized by other members of the MMP family. The cleavage of the cancer-associated protease site may be inhibided by inhibitors of gelatinase A and/or gelatinase B.

Further described is a method of inhibiting or destroying cancer cells, which cells are associated with a cancer-associated protease, comprising the steps of preparing an immunotoxin of the present invention having a furin site replaced by a cleavage site for a cancer-associated protease and administering the immunotoxin to the cells. In an embodiment, the cancer is selected from the group consisting of colorectal cancer, breast cancer, ovarian cancer, pancreatic cancer, head and neck cancer, bladder cancer, gastrointestinal cancer, prostate cancer, small cell and non small cell lung cancer, sarcomas, gliomas, T- and B-cell lymphomas.

The invention also includes the use of an effective amount of an immuntoxin of the invention for the manufacture of a medicament to treat a mammal with cancer. In one embodiment, the cancer cells are associated with a cancer-associated protease.

Still further, a process is described for preparing a pharmaceutical for treating a mammal with cancer wherein cells affected by the cancer are associated with a cancer-associated protease, comprising the steps of identifying a cleavage recognition site for the protease; preparing an Ep-CAM-targeted-ETA immunotoxin having the furin site, RQPR, replaced with the cancer-associated protease site and suspending the protein in a pharmaceutically acceptable carrier, diluent or excipient.

In a further aspect, the invention provides a pharmaceutical composition for treating a mammal with cancer wherein cells affected by the cancer are associated with a cancer-associated protease, comprising the immunotoxin of the invention and a pharmaceutically acceptable carrier, diluent or excipient.

Other features and advantages of the present invention will become apparent from the following detailed description.

### Brief description of the drawings

The invention will now be described in relation to the drawings in which:
**Figure 1** shows (a) a schematic representation of the proteolytic cleavage consensus site of the parental immunotoxin VB4-845, the MMP-processed IMMPA and a control immunotoxin (IMMDF). Arrows indicate the cleavage site in the furin consensus RQPR, which was replaced by the gelatinase substrate sequence GPLGMLSQ in IMMPA or by a sequence not sensitive to both proteases in IMMDF; (b) 3-D structure of IMMPA showing location of the cleavage point; (c) schematic of domain structure and location of cleavage site.
**Figure 2** shows the analysis of (a) VB4-845 and (b) IMMPA after *in vitro* cleavage. The immunotoxins were incubated with furin or the gelatinases MMP-2 and MMP-9 for the indicated time points, and samples were analyzed by polyacrylamide gel electrophoresis.
**Figure 3** shows a panel of EpCAM expression levels of tumor and immortalized normal cells after staining and FACS analysis.
**Figure 4** is a graphical representation of the inhibition of tumor cell lines overexpressing EpCAM.
**Figure 5** is a graphical representation of the cytotoxicity screening of other tumor cell lines and some immortalized normal cells.
**Figure 6** shows the expression of EpCAM in transfected cells by FACS analysis.
**Figure 7** is a graphical representation of the cytotoxicity of transfected cell lines.
**Figure 8** is a graphical representation of the effect of IMMPA on MCF-7 tumor cells after different incubation times. The cells were incubated with toxin for different time periods and then the supernatant was replaced by normal medium to follow the standard procedure of 72 h incubation used to evaluated viability in vitro (MTT). The graphic representation of 72 h incubation (black square with yellow cross) reflects the normal protocol where the toxin was never removed from the culture medium.

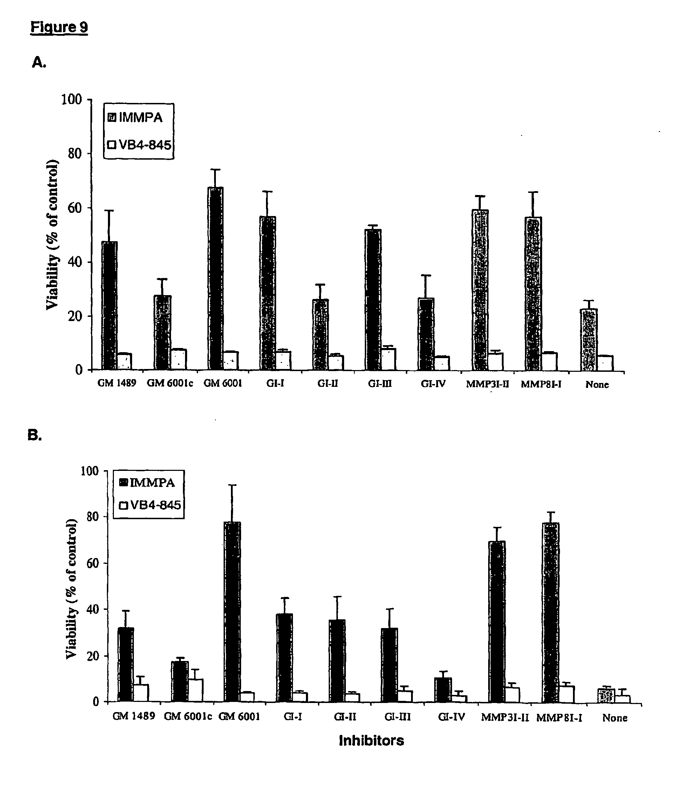

**Figure 9** is a graphical representation of the effect of MMP-inhibitors on activation of IMMPA and cell viability in MCF-7 and HT29 cells. The MMP inhibitors prevented the activation of IMMPA and inhibited death induction in MCF-7 cells. (a) 3'000 MCF-7 or (b) HT29 carcinoma cells were cultured overnight in 96-well plates in complete RPMI cell culture medium. The various MMP inhibitors were added at concentration of 100 µM for the MMP-2/MMP-9 inhibitors GM1489, GM6001, GI-I to IV or the control GM6001c, or 10µM for the MMP-3 and MMP-8 inhibitors in serum-free RPMI. Upon a 30 min preincubation, 100pM of immunotoxin IMMPA or VB4-845 was added and upon further 3 h of incubation the medium containing toxins and MMP inhibitors was replaced by complete RPMI before cells were incubated for 72 h in MTT viability assays. Cell viability is expressed as percentage relative to the viability of cells grown in the presence of the respective MMP inhibitors alone, which was arbitrarily set to 100%. Bars represent the mean of 3 independent experiments.
**Figure 10** is a graphical representation of cell viability measured in MTT assays.

The inventors have found that replacement of the furin site with the (gelatinase; MMP-2/MMP-9) protease-sensitive site in the immunotoxin VB4-845 increases the tissue-specificity of the cytotoxic effect of the immunotoxin, thus reducing much of the toxicity to normal tissue compared to wild type VB4-845 immunotoxin.

### Modified toxins of the invention

The invention provides a modified toxin comprising an ETA moiety having the furin site replaced with a cancer-associated protease site.

The term "ETA moiety" as used herein means exotoxin A from *Pseudomonas aeruginosa.* The term includes full length ETA as well as fragments or variants thereof that contain a furin site that can be replaced with a cancer-associated protease site.

In one embodiment, the furin site comprises the sequence RQPR. In another embodiment, the cancer-associated protease site is recognized by MMP-2, MMP-9 or a combination thereof. In yet another embodiment, the cancer-associated protease site comprises the sequence selected from the group consisting of GPLGMLSQ and GPLGLWAQ. In another embodiment, the cancer-associated protease site comprises more amino acids than the replaced furin site RQPR.

### Immunotoxins of the Invention

As previously mentioned, the present invention provides an immunotoxin comprising (a) an antibody or antibody fragment that binds to Ep-CAM on the surface of a cancer cell ; (b) a modified toxin comprising an ETA moiety that has the furin site replaced with a cancer-associated protease site. In one embodiment, the immunotoxin is internalized by the cancer cell.

When used in an immunotoxin, the "ETA moiety" can be a full length ETA or a fragment or variant thereof that contains a sufficient portion of ETA to be toxic to cancer cells. A variety of ETA toxins may be used to design an immunotoxin according to the invention. In preferred embodiments, the ETA toxin comprises at least a toxic portion of *Pseudomonas* exotoxin A ("ETA"), or a variant thereof in which the furin site is replaced by a cancer-associated protease site. In a specific embodiment, the cytotoxic portion comprises an ETA variant that, when administered alone, is substantially unable to bind to cells. The cytotoxic portion may comprise one or more *Pseudomonas* exotoxins known in the art (see, e.g., Kreitman, 1995, "Targeting pseudomonas exotoxin to hematologic malignancies," Seminars in Cancer Biology 6: 297-306; Pastan, 2003, "Immunotoxins containing pseudomonas exotoxin A: a short history," Cancer Immunol. Immunother. 52: 338-341), or variants thereof, in which the furin site is replaced by a cancer-associated protease site.

Several variants of Pseudomonas exotoxin, as well as methods of making and using constructs comprising Pseudomonas exotoxin variants, are known in the art (see, e.g., U.S. Patent Application No. US2003054012; U.S. Patent No. 6531133; U.S. Patent No. 6426075; U.S. Patent No. 6423513; U.S. Patent No. 6074644; U.S. Patent No. 5980895; U.S. Patent No. 5912322; U.S. Patent No. 5854044; U.S. Patent No. 5821238; U.S. Patent No. 5705163; U.S. Patent No. 5705156; U.S. Patent No. 5621078; U.S. Patent No. 5602095; U.S. Patent No. 5512658; U.S. Patent No. 5458878; U.S. Patent No. 5082927; U.S. Patent No. 4933288; U.S. Patent No. 4892827; U.S. Patent No. 4677070; U.S. Patent No. 4545985; International Publication Nos. WO98/20135, WO93/25690; WO91/18100; WO91/18099; WO91/09949; and WO88/02401; Kondo et al., 19888, "Activity of immunotoxins constructed with modified pseudomonas exotoxin a lacking the cell recognition domain." J Biol Chem. 263:9470-9475; Batra et al., 1989, "Antitumor activity in mice of an immunotoxin made with anti-transferring receptor and a recombinant form of pseudomonas exotoxin." Proc Natl.Acad.Sci.USA 86:8545-8549; Puri et al., 1991, "Expression of high-affinity interleukin 4 receptors on murine sarcoma cells and receptor-mediated cytotoxicity of tumor cells to chimeric protein between interleukin 4 and Pseudomonas exotoxin." Cancer Res 51:3011-3017; Siegall et al., 1992, "Cytotoxicity of chimeric (human murine) monoclonal antibody BR96 IgG, F(ab')2, and Fab' conjugated to Pseudomonas exotoxin." Bioconjug-Chem 3:302-307; Hall et al., 1994, "In vivo efficacy of intrathecal transferrin-Pseudomonas exotoxin A immunotoxin against LOX melanoma." Neurosurgery 34:649-655; Kuan and Pai, 1995, "Immunotoxins containing pseudomonas exotoxin that target Le y damage human endothelial cells in an antibody-specific mode: relevance to vascular leak syndrome." Clin Cancer Res 1:1589-1594; Kreitman, 1995, "Targeting pseudomonas exotoxin to hematologic malignancies." Sem Cancer Biol 6:297-306; Kawooya et al., "The expression, affinity purification and characterization of recombinant pseudomonas exotoxin 40 (PE40) secreted from Escherichia coli." J Biotechnol 42:9-22; Kaun and Pai, 1995, "Immunotoxins containing pseudomonas exotoxin that target LeY damage human endothelial cells in an antibody-specific mode: Relevance to vascular leak syndrome." Clin Cancer Res 1:1589-1594; Puri et al., 1996, "Preclinical development of a recombinant toxin containing circularly permuted interleukin 4 and truncated Pseudomonas exotoxin for therapy of malignant astrocytoma." Cancer Res 56:5631-5637; Pai et al., 1996, "Treatment of advanced solid tumors with immunotoxin LMB-1: An antibody linked to Pseudomonas exotoxin." Nature Med. 3:350-353; Pai et al., 1998, "Clinical Trials with pseudomonas exotoxin immunotoxins." Curr Top. Microbiol. Immunol. 234: 83-96; Klimka et al., 1999, "An anti-CD30 single chain Fv selected by phage display and fused to pseudomonas exotoxin A (Ki-4(scFv)-ETA') is a potent immunotoxin against a Hodgkin-derived cell line." British J Cancer 80:1214-1222; Rand et al., 2000, "Intratumoral administration of recombinant circularly permuted interleukin-4-Pseudomonas exotoxin in patients with high-grade glioma." Clin Cancer Res 6:2157-2165; Leland et al., 2000, "Human breast carcinoma cells express type II IL-4 receptors and are sensitive to antitumor activity of chimeric IL-4-pseudomonas exotoxin fusion protein in vitro and in vivo." Molecular Medicine Today 6:165-178;Tur et al., 2001, "An anti-GD2 single chain Fv selected by phage display and fused to Pseudomonas exotoxin A develops specific cytotoxic activity against neuroblastoma derived cell lines." Int J Mol.Med 8:579-584; Onda et al., 2001, "Cytotoxicity of antiosteosarcoma recombinant immunotoxins composed of TP-3 Fv fragments and a truncated pseudomonas exotoxin A." J Immunother 24:144-150; 18. "Synergistic interaction between an anti-p185her-2 pseudomonas exotoxin fusion protein [scfv(frp5)-eta] and ionizing radiation for inhibiting growth of ovarian cancer cells that overexpress HER-2." Schmidt et al., 2001, "Synergistic interaction between an anti-p185HER-2 pseudomonas exotoxin fusion protein [scFv(FRP5)-ETA] and ionizing radiation for inhibiting growth of ovarian cancer cells that overexpress HER-2. "Gynecol Oncol 80:145-155; Pastan, 2003, "immunotoxins containing pseudomonas exotoxin A: a short history." Cancer Immunol Immunother 52:338-341; Li et al., 1996, "Crystal structure of the catalytic domain of Pseudomonas exotoxin A complexed with a nicotinamide adenine dinucleotide analog: implications for the activation process and for ADP ribosylation." Proc Natl Acad Sci USA. 9:6902-6906; Kreitman and Pastan, 2003, "Immunobiological treatments of hairy-cell leukaemia." Best Pract Res Clin Haematol. 16:117-33.

Component (a) of the immunotoxin is an antibody or antibody fragment that recognizes Ep-CAM on the surface of a cancer cell. In a preferred embodiment, the antibody is internalized by the cancer cell.

In a most particular embodiment, the present invention provides an immunotoxin comprising a mutated VB4-845 immunotoxin having the furin site of the ETA moiety replaced with a cancer-associated protease site. The term "VB4-845" as used in herein means an immunotoxin that comprises a) the scFv humanized antibody 4D5MOC-B that is fused to b) a truncated form of ETA that consists of amino acids 252-608 as well as a HIS tag and the KDEL sequence.

In one embodiment, the furin site comprises the sequence RQPR. The cancer-associated protease site is recognized by MMP-2, MMP-9 or a combination thereof. In another embodiment, the cancer-associated protease site comprises the sequence selected from the group consisting of GPLGMLSQ and GPLGLWAQ. In another embodiment, the cancer-associated protease site comprises more amino acids than the replaced furin site RQPR. It is an advantage of the immunotoxins of the invention that they are non-toxic until the protease site is cleaved by the target protease.

The immunotoxins of the present invention may be used to treat various forms of cancer such as colorectal cancer, breast cancer, ovarian cancer, pancreatic cancer, head and neck cancer, bladder cancer, gastrointestinal cancer, prostate cancer, small cell and non small cell lung cancer, sarcomas, gliomas, T- and B-cell lymphomas.

Accordingly, in one embodiment, the invention provides an Ep-CAM-targeted-ETA immunotoxin comprising (a) an antibody or antibody fragment that binds to Ep-CAM on the cancer cell attached to; (b) a modified toxin comprising an ETA moiety that has the furin site replaced with a cancer-associated protease site. In a specific embodiment, the immunotoxin comprises (a) a humanized antibody or antibody fragment that binds to the extracellular domain of human Ep-CAM and comprises complementarity determining region (CDR) sequences derived from a MOC-31 antibody attached to: (b) a modified toxin comprising an ETA moiety that has the furin site replaced with a cancer-associated protease site.

Suitable Ep-CAM-targeted-ETA immunotoxins according to the invention include, without limitation, VB4-845 and variants thereof, other immunotoxins that comprises other single or double chain immunoglobulins that selectively bind Ep-CAM, or variants thereof.

Thus the immunotoxin may be used to specifically target cancer cells. It is a further advantage that the cancer-associated protease cleaves the immunotoxin intracellularly thereby allowing efficient trafficking of the toxin and ultimately resulting in cell death. As a result, said cancer cells are specifically targeted and normal cells that do not contain the cancer-associated protease are not directly exposed to the activated ETA.

In one embodiment, the Ep-CAM-binding portion comprises a complete immunoglobulin molecule. In another embodiment, the Ep-CAM-binding portion is a dimer of Fab, Fab', scfv, single-domain antibody fragments, or disulfide-stabilized Fv fragments. In another embodiment, the Ep-CAM-binding portion comprises a variable heavy chain, variable light chain, Fab, Fab', scfv, single-domain antibody fragment, or disulfide-stabilized Fv fragment. Portions of the Ep-CAM-binding molecule may be derived from one or more species, preferably comprising portions derived from the human species, and most preferably are completely human or humanized. Regions designed to facilitate purification or for conjugation to toxin may also be included in or added to the Ep-CAM-binding portion.

In a specific, non-limiting embodiment, the immunotoxin comprises VB4-845. In other non-limiting embodiments, the immunotoxin comprises a variant of VB4-845. A VB4-845 variant binds to the same Ep-CAM epitope or to a substantially similar Ep-CAM epitope that is bound by VB4-845, and the variant may competitively inhibit VB4-845 binding to Ep-CAM, under physiologic conditions, by at least 10%, 15%, 20%, 25%, 30%, 35%, 40%, 45%, 50%, 55%, 60%, 65%, 70%, 75%, 80%, 85%, 90%, or 95%. A VB4-845 variant may comprise the same Pseudomonas exotoxin A fragment as VB4-845, or may comprise a different portion of the same exotoxin or a different toxin.

In another non-limiting embodiment, the immunotoxin comprises an Ep-CAM-binding portion comprising the variable region of MOC31, or a variant thereof. In yet another embodiment, the immunotoxin comprises an Ep-CAM-binding portion comprising 4D5MOCB, or a variant thereof. Binding of any of these immunotoxins to Ep-CAM may be reduced by at least 10%, 15%, 20%, 25%, 30%, 35%, 40%, 45%, 50%, 55%, 60%, 65%, 70%, 75%, 80%, 85%, 90%, or 95% by competition with the reference MOC31 or 4D5MOCB antibody under physiologic conditions. The affinity of VB4-845 is K_{D}=1.6 x10⁻⁸, using indirect flow cytometry on live cells. Lineweaver-Burke analysis (data Notebook: 0935, page 50) was performed using method of Benedict et al (1997). J. Immunol. Methods, 201:223-231. The affinity of MOC31 B , as described in Willuda et al (Cancer Research 59, 5758-5767, 1999) is K_{D} = 3.9 x 10⁻⁹, measured using RIA and Biacore as described in methods. Consequently, the present invention includes immunotoxins having a dissociation constant (K_{D}) of less than 2.0 x 10⁻⁸.

Alternatively, the immunotoxin comprises an Ep-CAM-binding portion other than those discussed in the preceding paragraphs, but which selectively binds to Ep-CAM. In a preferred embodiment, the binding affinity of said Ep-CAM-binding portion is at least four orders of magnitude, preferably at least three orders of magnitude, more preferably less than two orders of magnitude of the binding affinity of VB4-845, PANOREX®, or MT-201 as measured by standard laboratory techniques. In non-limiting embodiments, the Ep-CAM-binding portion may competitively block the binding of a known anti-Ep-CAM antibody, such as, but not limited to, PANOREX® or MT201, to Ep-CAM, under physiologic conditions, by at least 0.1%, 1%, 10%, 15%, 20%, 25%, 30%, 35%, 40%, 45%, 50%, 55%, 60%, 65%, 70%, 75%, 80%, 85%, 90%, or 95%.

The skilled artisan would appreciate that binding regions of the antibody, such as CDR or hot-spots can be identified. Individual substitutions of amino acids in the binding region, such as with alanine, or other mutations can diminish the affinity of the antibody for the epitope by at least 10 fold, preferably by at least 100 fold, more preferably by at least 1000 fold. This loss in affinity underscores that residue's importance in the ability of the antibody to bind the epitope. See, e.g., Tamura et al., 2000, "Structural correlates of an anticarcinoma antibody: identification of specificity-determining residues (SDRs) and development of a minimally immunogenic antibody variant by retention of SDRs only," J. Immunol. 164(3):1432-1441.

The effect of single or multiple mutations on binding activity, particularly on binding affinity, may be evaluated contemporaneously to assess the importance of a particular series of amino acids on the binding interaction (e.g., the contribution of the light or heavy chain CDR2 to binding). Effects of an amino acid mutation may also be evaluated sequentially to assess the contribution of a single amino acid when assessed individually. Such evaluations can be performed, for example, by in vitro saturation scanning (see, e.g., U.S. Patent No. 6,180,341; Hilton et al., 1996, "Saturation mutagenesis of the WSXWS motif of the erythropoietin receptor," J Biol Chem. 271:4699-4708) and site-directed mutagenesis (see, e.g., Cunningham and Wells, 1989, "High-resolution epitope mapping of hGH-receptor interactions by alanine-scanning mutagenesis," Science 244:1081-1085; Bass et al., 1991, "A systematic mutational analysis of hormone-binding determinants in the human growth hormone receptor," Proc Natl Acad Sci. USA 88:4498-4502). In the alanine-scanning mutagenesis technique, single alanine mutations are introduced at multiple residues in the molecule, and the resultant mutant molecules are tested for biological activity to identify amino acid residues that are critical to the activity of the molecule.

Sites of ligand-receptor or other biological interaction can also be identified by physical analysis of structure as determined by, for example, nuclear magnetic resonance, crystallography, electron diffraction, or photoaffinity labeling, in conjunction with mutation of putative contact site amino acids (see, e.g., de Vos et al., 1992, "Human growth hormone and extracellular domain of its receptor: crystal structure of the complex," Science 255:306-312; Smith et al., 1992, "Human interleukin 4. The solution structure of a four-helix bundle protein," J Mol Biol. 224:899-904; Wlodaver et al., 1992, "Crystal structure of human recombinant interleukin-4 at 2.25 A resolution," FEBS Lett. 309:59-64. Additionally, the importance of particular individual amino acids, or series of amino acids, may be evaluated by comparison with the amino acid sequence of related polypeptides or analogous binding sites.

Furthermore, the skilled artisan would appreciate that increased avidity may compensate for lower binding affinity. The avidity of an immunotoxin for Ep-CAM is an measure of the strength of the Ep-CAM-binding portion's binding of Ep-CAM, which has multiple binding sites. The functional binding strength between Ep-CAM and the Ep-CAM-binding portion represents the sum strength of all the affinity bonds, and thus an individual component may bind with relatively low affinity, but a multimer of such components may demonstrate potent biological effect. In fact, the multiple interactions between Ep-CAM-binding sites and Ep-CAM epitopes may demonstrate much greater than additive biological effect, i.e., the advantage of multivalence can be many orders of magnitude with respect to the equilibrium constant.

In one non-limiting embodiment, the Ep-CAM-binding portion has a structure substantially similar to that of 4D5MOCB. The substantially similar structure can be characterized by reference to epitope maps that reflect the binding points of the immunotoxin's Ep-CAM-binding portion to an Ep-CAM molecule.

The immunotoxins of the present invention may be prepared by chemical synthesis using techniques well known in the chemistry of proteins such as solid phase synthesis (Merrifield, J. Am. Chem. Assoc. 85:2149-2154 (1964)) or synthesis in homogenous solution (Houbenweyl, Methods of Organic Chemistry, ed. E. Wansch, Vol. 15 I and II, Thieme, Stuttgart (1987)). In one embodiment, the cancer-binding antibody and ETA toxin are both proteins and can be conjugated using techniques well known in the art. There are several hundred crosslinkers available that can conjugate two proteins. (See for example "Chemistry of Protein Conjugation and Crosslinking". 1991, Shans Wong, CRC Press, Ann Arbor). The crosslinker is generally chosen based on the reactive functional groups available or inserted on the ligand or toxin. In addition, if there are no reactive groups a photoactivatible crosslinker can be used. In certain instances, it may be desirable to include a spacer between the ligand and the toxin. Crosslinking agents known to the art include the homobifunctional agents: glutaraldehyde, dimethyladipimidate and Bis(diazobenzidine) and the heterobifunctional agents: m Maleimidobenzoyl-N-Hydroxysuccinimide and Sulfo-*m* Maleimidobenzoyl-N-Hydroxysuccinimide.

A antibody ETA toxin fusion protein may also be prepared using recombinant DNA techniques. In such a case a DNA sequence encoding the cancer-binding antibody is fused to a DNA sequence encoding the mutated ETA toxin, resulting in a chimeric DNA molecule The chimeric DNA sequence is transfected into a host cell that expresses the antibody toxin fusion protein. The fusion protein can be recovered from the cell culture and purified using techniques known in the art.

Antibodies having specificity for tumour antigens such as Ep-CAM may be prepared by conventional methods. A mammal, (*e.g.* a mouse, hamster, or rabbit) can be immunized with an immunogenic form of the peptide which elicits an antibody response in the mammal. Techniques for conferring immunogenicity on a peptide include conjugation to carriers or other techniques well known in the art. For example, the peptide can be administered in the presence of adjuvant. The progress of immunization can be monitored by detection of antibody titers in plasma or serum. Standard ELISA or other immunoassay procedures can be used with the immunogen as antigen to assess the levels of antibodies. Following immunization, antisera can be obtained and, if desired, polyclonal antibodies isolated from the sera.

To produce monoclonal antibodies, antibody-producing cells (lymphocytes) can be harvested from an immunized animal and fused with myeloma cells by standard somatic cell fusion procedures thus immortalizing these cells and yielding hybridoma cells. Such techniques are well known in the art, (e.g. the hybridoma technique originally developed by Kohler and Milstein (Nature 256:495-497 (1975)) as well as other techniques such as the human B-cell hybridoma technique (Kozbor et al., Immunol. Today 4:72 (1983)), the EBV-hybridoma technique to produce human monoclonal antibodies (Cole et al., Monoclonal Antibodies in Cancer Therapy Allen R., Bliss, Inc., pages 77-96 (1985)), and screening of combinatorial antibody libraries (Huse et al., Science 246:1275 (1989)). Hybridoma cells can be screened immunochemically for production of antibodies specifically reactive with the peptide and the monoclonal antibodies can be isolated.

The term "antibody" as used herein is intended to include fragments thereof which also specifically react with a cell surface component. Antibodies can be fragmented using conventional techniques and the fragments screened for utility in the same manner as described above. For example, F(ab')2 fragments can be generated by treating antibody with pepsin. The resulting F(ab')2 fragment can be treated to reduce disulfide bridges to produce Fab' fragments.

Chimeric antibody derivatives, i.e., antibody molecules that combine a non-human animal variable region and a human constant region are also contemplated within the scope of the invention. Chimeric antibody molecules can include, for example, the antigen binding domain from an antibody of a mouse, rat, or other species, with human constant regions. Conventional methods may be used to make chimeric antibodies containing the immunoglobulin variable region which recognizes a cell surface antigen (See, for example, Morrison et al., Proc. Natl Acad. Sci. U.S.A. 81:6851 (1985); Takeda et al., Nature 314:452 (1985), Cabilly et al., U.S. Patent No. 4,816,567; Boss et al., U.S. Patent No. 4,816,397; Tanaguchi et al., E.P. Patent No. 171,496; European Patent No. 173,494, United Kingdom Patent No. GB 21770968). It is expected that chimeric antibodies would be less immunogenic in a human subject than the corresponding non-chimeric antibody. Chimeric antibodies can be stabilized by the method described in Pluckthun et al., WO 00/61634.

Monoclonal or chimeric antibodies specifically reactive against cell surface components can be further humanized by producing human constant region chimeras, in which parts of the variable regions, particularly the conserved framework regions of the antigen-binding domain, are of human origin and only the hypervariable regions are of non-human origin. Such immunoglobulin molecules may be made by techniques known in the art, (e.g. Teng et al., Proc. Natl. Acad. Sci. U.S.A., 80:7308-7312 (1983); Kozbor et al., Immunology Today 4:7279 (1983); Olsson et al., Meth. Enzymol., 92:3-16 (1982), and PCT Publication WO92/06193 or EP 239,400). Humanized antibodies can also be commercially produced (Scotgen Limited, 2 Holly Road, Twickenham, Middlesex, Great Britain.)

Specific antibodies, or antibody fragments, reactive against cell surface components may also be generated by screening expression libraries encoding immunoglobulin genes, or portions thereof, expressed in bacteria with cell surface components. For example, complete Fab fragments, VH regions and FV regions can be expressed in bacteria using phage expression libraries (See for example Ward et al., Nature 341:544-546 (1989); Huse et al., Science 246:1275-1281 (1989); and McCafferty et al., Nature 348:552-554 (1990)). Alternatively, a SCID-hu mouse, for example the model developed by Genpharm, can be used to produce antibodies, or fragments thereof.

In one embodiment, the antibody is internalized by a cancer cell.

### Utility of the Immunotoxins of the Invention

The proteins of the invention may be used to specifically inhibit or destroy mammalian cells affected by cancer which have associated with such cells a protease. It is an advantage of the immunotoxins of the invention that they have specificity for said cells in addition to that gained by the cell-binding component. The cancer-associated protease site of the ETA moiety is exposed once the molecule unfurls inside the endosome due to a lower pH, thus, in the presence of the cancer-associated protease, the ETA toxin is released allowing for efficient trafficking of the toxin and ultimately resulting in cell death.

Accordingly, a method of inhibiting or destroying cancer cells is provided which cells are associated with a cancer-associated protease, comprising the steps of preparing an immunotoxin of the present invention having a furin site replaced by a cleavage site for a cancer-associated protease and administering the immunotoxin to the cells. In an embodiment, the cancer is colorectal cancer, breast cancer, ovarian cancer, pancreatic cancer, head and neck cancer, bladder cancer, gastrointestinal cancer, prostate cancer, small cell and non small cell lung cancer, sarcomas, gliomas, T- and B-cell lymphomas.

The specificity of an immunotoxin of the invention may be tested by treating the immunotoxin with the cancer-associated protease which is thought to be specific for the cleavage recognition site and assaying for cleavage products. Cancer-associated proteases may be isolated from cancer cells or they may be prepared recombinantly, for example following the procedures in Darket et al. (J. Biol. Chem. 254:2307-2312 (1988)). The cleavage products may be identified for example based on size, antigenicity or activity. The toxicity of the immunotoxin may be investigated by subjecting the cleavage products to an *in vitro* translation assay in cell lysates, for example using Brome Mosaic Virus mRNA as a template. Toxicity of the cleavage products may be determined using a ribosomal inactivation assay (Westby et al., Bioconjugate Chem. 3:377-382 (1992)). The effect of the cleavage products on protein synthesis may be measured in standardized assays of *in vitro* translation utilizing partially defined cell free systems composed for example of a reticulocyte lysate preparation as a source of ribosomes and various essential cofactors, such as mRNA template and amino acids. Use of radiolabelled amino acids in the mixture allows quantitation of incorporation of free amino acid precursors into trichloroacetic acid precipitable proteins. Rabbit reticulocyte lysates may be conveniently used (O'Hare, FEBS Lett. 273:200-204 (1990)).

The ability of the immunotoxins of the invention to selectively inhibit or destroy animal cancer cells may be readily tested *in vitro* using animal cancer cell lines. The selective inhibitory effect of the immunotoxins of the invention may be determined, for example, by demonstrating the selective inhibition of cellular proliferation in cancer cells. In addition, the protease specificity can be tested by comparing the inhibition of cellular proliferation using an immunotoxins of the invention alone or in the presence of protease-specific inhibitors. Such protease inhibitors may include MMP-2/MMP-9 inhibitors GM1489, GM6001 and GI-I to GI-IV.

Toxicity may also be measured based on cell viability, for example the viability of normal and cancerous cell cultures exposed to the immunotoxins may be compared. Cell viability may be assessed by known techniques, such as trypan blue exclusion assays.

In another example, a number of models may be used to test the cytotoxicity of immunotoxins having a cancer-associated protease sequence containing a cleavage recognition site for a cancer-associated matrix metalloprotease. Thompson, E.W. et al. (Breast Cancer Res. Treatment 31:357-370 (1994)) has described a model for the determination of invasiveness of human breast cancer cells *in vitro* by measuring tumour cell-mediated proteolysis of extracellular matrix and tumour cell invasion of reconstituted basement membrane (collagen, laminin, fibronectin, Matrigel or gelatin). Other applicable cancer cell models include cultured ovarian adenocarcinoma cells (Young, T.N. et al. Gynecol. Oncol. 62:89-99 (1996); Moore, D.H. et al. Gynecol. Oncol. 65:78-82 (1997)), human follicular thyroid cancer cells (Demeure, M.J. et al., World J. Surg. 16:770-776 (1992)), human melanoma (A-2058) and fibrosarcoma (HT-1080) cell lines (Mackay, A.R. et al. Lab. Invest. 70:781-783 (1994)), and lung squamous (HS-24) and adenocarcinoma (SB-3) cell lines (Spiess, E. et al. J. Histochem. Cytochem. 42:917-929 (1994)). An *in vivo* test system involving the implantation of tumours and measurement of tumour growth and metastasis in athymic nude mice has also been described (Thompson, E.W. et al., Breast Cancer Res. Treatment 31:357-370 (1994); Shi, Y.E. et al., Cancer Res. 53:1409-1415(1993)).

Also described is method of treating a mammal with cancer wherein cells affected by the cancer are associated with a cancer-associated protease by administering an effective amount of one or more immunotoxins of the present invention to said mammal. The term "treating a mammal with cancer" as used herein refers to inhibiting cancer cell replication, inhibiting cancer spread (metastasis), inhibiting tumor growth, reducing cancer cell number or tumor growth, decreasing the malignant grade of a cancer (e.g., increased differentiation), or improving cancer-related symptoms in the mammal.

The invention also includes the use of an effective amount of an immuntoxin of the invention for the manufacture of a medicament to treat a mammal with cancer. In one embodiment, the cancer cells are associated with a cancer-associated protease.

Further described is a process is for preparing a pharmaceutical for treating a mammal with cancer wherein cells affected by the cancer are associated with a cancer-associated protease, comprising the steps of identifying a cleavage recognition site for the protease; preparing an immunotoxin of the invention having the furin site replaced with the cancer-associated protease site and suspending the protein in a pharmaceutically acceptable carrier, diluent or excipient.

The invention also provides a pharmaceutical composition for treating a mammal with cancer wherein cells affected by the cancer are associated with a cancer-associated protease comprising an immunotoxin of the invention and a pharmaceutically acceptable carrier, diluent or excipient.

In a preferred embodiment, the mammal is human. The cancer-associated protease recognizes the MMP-2, MMP-9 cleavage sites or a combination thereof. In another embodiment, the cancer is selected from the group consisting of colorectal cancer, breast cancer, ovarian cancer, pancreatic cancer, head and neck cancer, bladder cancer, gastrointestinal cancer, prostate cancer, small cell and non small cell lung cancer, sarcomas, gliomas and T- and B-cell lymphomas.

The immunotoxins of the invention may be formulated into pharmaceutical compositions for administration to subjects in a biologically compatible form suitable for administration *in vivo.* By "biologically compatible form suitable for administration in vivo" is meant a form of the substance to be administered in which any toxic effects are outweighed by the therapeutic effects. The substances may be administered to living organisms including humans, and animals. Administration of a therapeutically active amount of the pharmaceutical compositions of the present invention or "effective amount" of the pharmaceutical compositions of the present invention means as an amount effective, at dosages and for periods of time necessary to achieve the desired result. For example, a therapeutically active amount of a substance may vary according to factors such as the disease state, age, sex, and weight of the individual, and the ability of antibody to elicit a desired response in the individual. Dosage regime may be adjusted to provide the optimum therapeutic response. For example, several divided doses may be administered daily or the dose may be proportionally reduced as indicated by the exigencies of the therapeutic situation.

The active substance may be administered in a convenient manner such as by injection (subcutaneous, intravenous, intramuscular, etc.), oral administration, inhalation, transdermal administration (such as topical cream or ointment, etc.), or suppository applications. Depending on the route of administration, the active substance may be coated in a material to protect the compound from the action of enzymes, acids and other natural conditions which may inactivate the compound.

The compositions described herein can be prepared by *per se* known methods for the preparation of pharmaceutically acceptable compositions which can be administered to subjects, such that an effective quantity of the active substance is combined in a mixture with a pharmaceutically acceptable vehicle. Suitable vehicles are described, for example, in Remington's Pharmaceutical Sciences (Remington's Pharmaceutical Sciences, 20th ed., Mack Publishing Company, Easton, Pa., USA 2000). On this basis, the compositions include, albeit not exclusively, solutions of the substances in association with one or more pharmaceutically acceptable vehicles or diluents, and contained in buffered solutions with a suitable pH and iso-osmotic with the physiological fluids.

The pharmaceutical compositions may be used in methods for treating animals, including mammals, preferably humans, with cancer. It is anticipated that the compositions will be particularly useful for treating patients with colorectal cancer, breast cancer, ovarian cancer, pancreatic cancer, head and neck cancer, bladder cancer, gastrointestinal cancer, prostate cancer, small cell and non small cell lung cancer, sarcomas, gliomas, T- and B-cell lymphomas. The dosage and type of immunotoxin to be administered will depend on a variety of factors which may be readily monitored in human subjects. Such factors include the etiology and severity (grade and stage) of neoplasia.

As mentioned above, the novel immunotoxins of the present invention are useful in treating cancerous cells wherein the cells contain a protease that can cleave the cancer-associated cleavage site of the immunotoxin. One skilled in the art can appreciate that many different immunotoxins can be prepared once a cancer associated protease has been identified.

The following non-limiting examples are illustrative of the present invention:

### Example 1:

### Immunotoxin containing an MMP-specific Cleavable Linker

The recombinant single-chain immunotoxin 4D5MOCB-ETA (VB4-845) effectively kills human tumor cells expressing the carcinoma-associated antigen Ep-CAM. Upon Ep-CAM binding on the cell surface VB4-845 is internalized by receptor-mediated endocytosis and the ETA portion is subsequently released into the cytosol upon processing by furin, a protease ubiquitously expressed in mammalian cells.

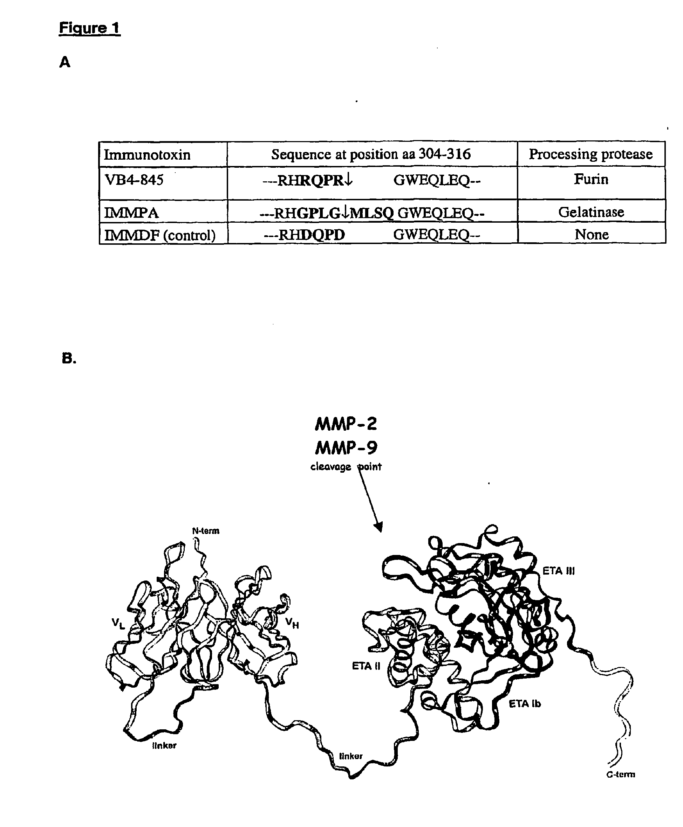

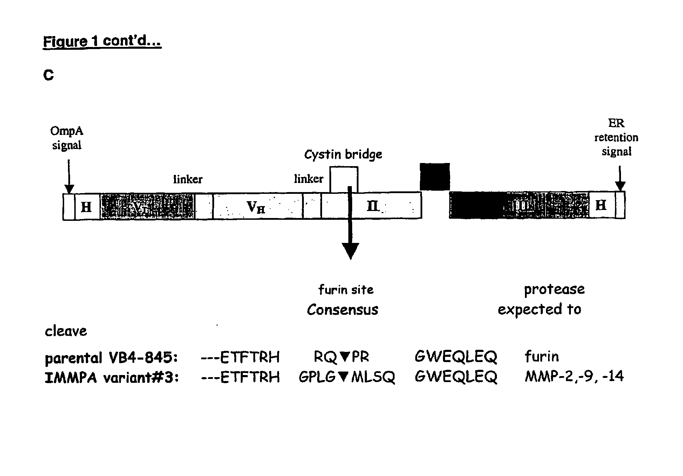

A new "pro-drug" like immunotoxin variant (IMMPA) was generated by replacing the furin consensus recognition sequence RQPR, present at position aa 306-309 of the wild-type toxin, with the cleavage site GPLGMLSQ recognized by a subclass of matrix metalloproteinases (MMPs) called gelatinase A (MMP-2) and gelatinase B (MMP-9) (Figure 1). Both proteases have been shown to be abundant in tumor tissues and their activity is upregulated during different stages of tumor development and malignant progression. As control, a cleavage site deficient immunotoxin variant (IMMDF) was developed, which is resistant to proteolytic cleavage by either furin or MMPs.

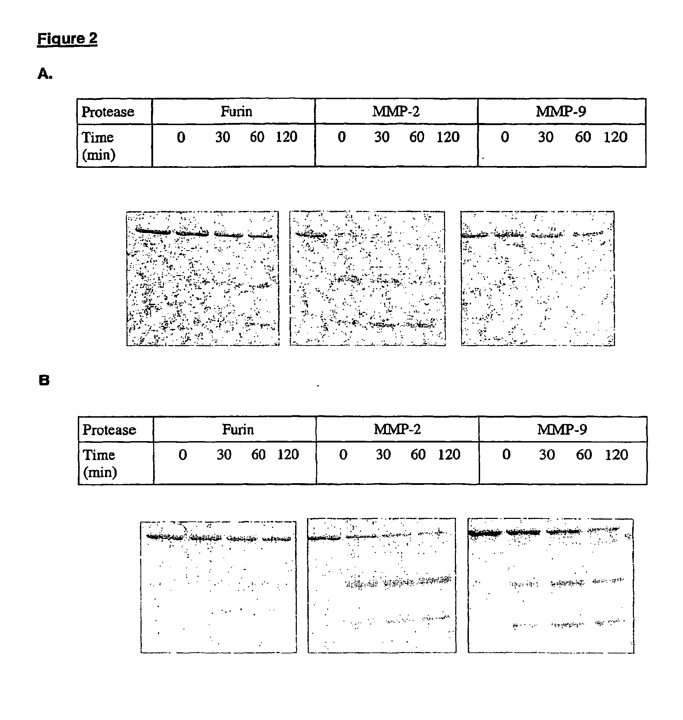

Upon Ep-CAM-mediated internalization into endo/lysosomes, the immunotoxin undergoes a conformational change that uncovers the protease recognition site and makes the toxin susceptible to proteolytic cleavage. This was confirmed for the parental immunotoxin VB4-845, which is hydrolyzed by furin and unexpectedly also by MMP-2 but not MMP-9 (Fig. 2). After mutation of the consensus site, proteolytic cleavage of IMMPA in vitro was restricted solely to the two activated gelatinases and was resistant to furin.

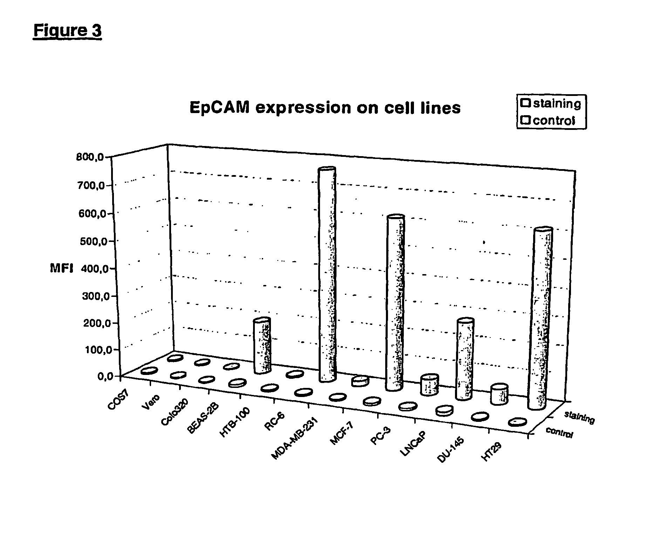

The expression of EpCAM on various cell lines was investigated. Figure 3 shows a panel of EpCAM expression levels of tumor and immortalised normal cells after staining and FACS analysis. A description focussing on EpCAM expression and MMP expression profile is listed in Table 1.

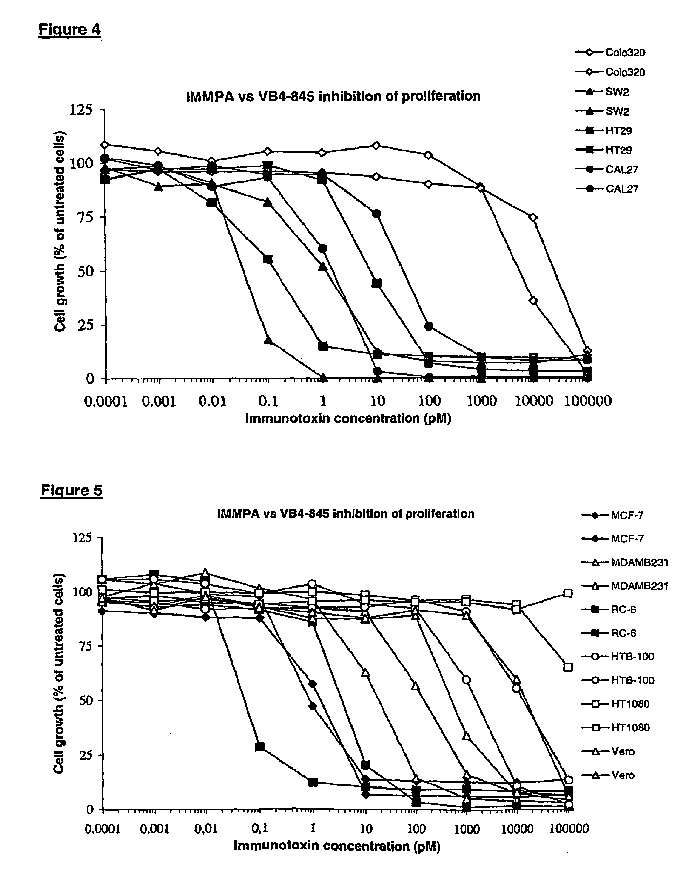

The proliferation of various tumor cell lines overexpressing EpCAM was assessed in the presence of either IMMPA or VB4-845 (Figure 4). The IMMPA is slightly less toxic, probably due to lower activity of MMP compared to furin, but also because IMMPA is processed by MMP only and not by both proteases. Nevertheless IMMPA is sufficiently toxic to be medically relevant. The cytotoxicity towards other tumor cell lines and some immortalised normal cells was also investigated in the presence of IMMPA or VB4-845 (Figure 5). Except for the breast carcinoma cell line MCF-7, which was similarly susceptible to IMMPA and VB4-845, the viability of all other cell lines was reduced by approximately one log. EpCAM-negative cell lines, such as Vero and HTB-100 were much less sensitive to VB4-845 than EpCAM-positive cells line , with HT1080 not affected even at a concentration of 100 nM and were even less affected by IMMPA.

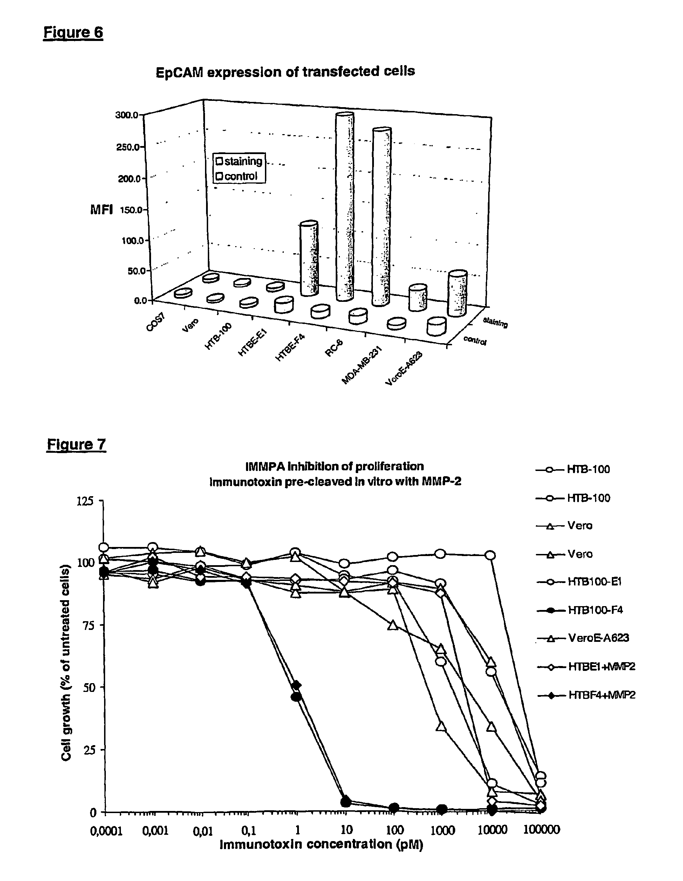

HTB100 and Vero were chosen as negative controls and transfected with a construct encoding for EpCAM. Protein expression was confirmed by FACS analysis (Figure 6). The results show the expression of a moderate (E1) and a high (F4) HTB100 producer cell clone.

The cytotoxicity of the transfected cell lines is shown in Figure 7. HTB100 high expresser was very sensitive to IMMPA activity, whereas the low expresser proliferated as the parental HTB100 cells. This indirectly demonstrated that HTB100 have MMP activity. Immortalisation using SV40 virus has been shown to influence MMP activity. The Vero EpCAM-expressing clone was shown to have only a low EPCAM expression level and, probably as a result of this low expression, was not sensitive to toxin treatment.

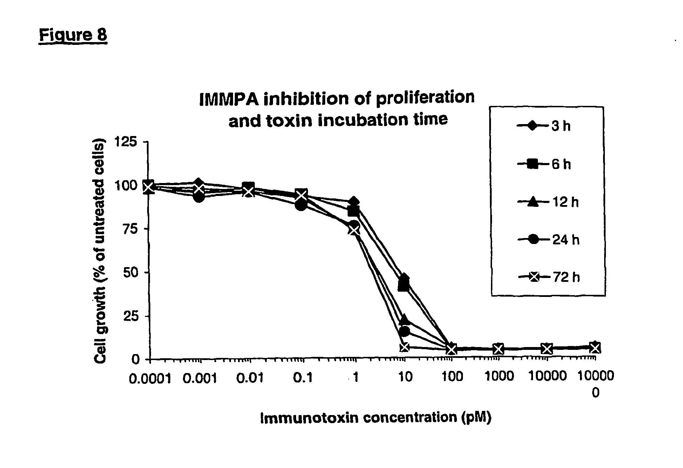

The effect of IMMPA on MCF-7 tumor cells after different incubation times was assessed (Figure 8). The viability of these cells was reduced to minimum at a concentration of 100 pM for all the schedules applied independently of the incubation time.

Having demonstrated the MMP-specific activation of IMMPA, its ability to reduce cell viability in an MMP-dependent manner was also assessed using a panel of MMP inhibitors (Figure 9). Ep-CAM-positive tumor cell lines (MCF-7 breast and HT29 colon carcinoma cells) were preincubated with a fixed dose of MMP-inhibitors and then treated with high dose IMMPA or parental VB4-845. As shown for both cell lines, the presence of MMP inhibitors prevented the full activation of IMMPA and significantly inhibited cell death induction, whereas no inhibition of the cytotoxic activity of VB4-845 was observed.

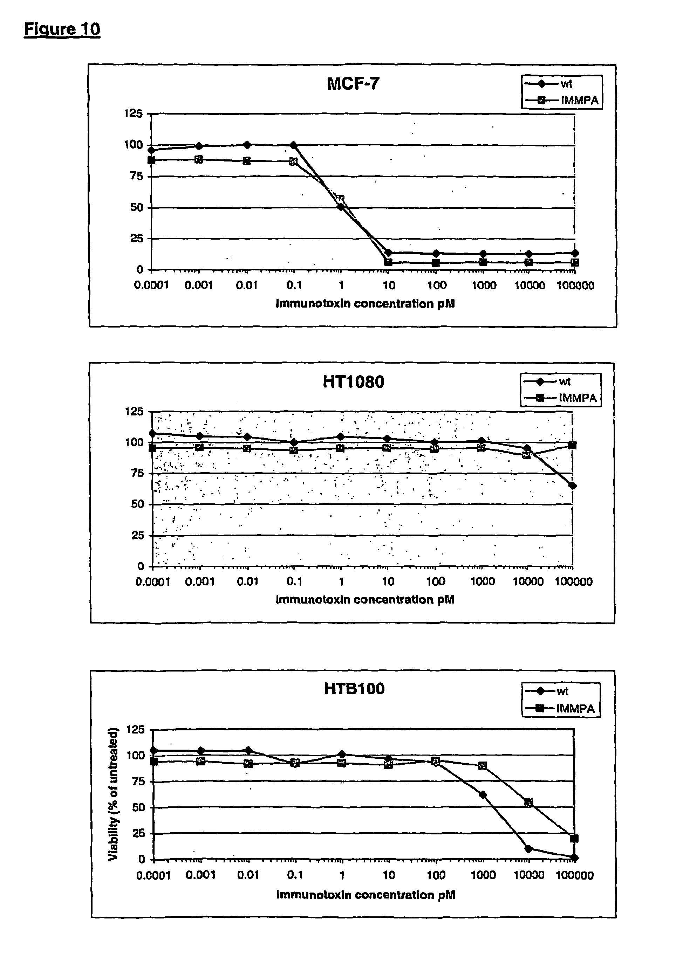

To further assess the specificity of the MMP-cleavable immunotoxin variant IMMPA for cancer cells that express MMPs, its cytotoxic activity against Ep-CAM-overexpressing MCF-7 breast carcinoma cells, the normal breast epithelial cell line HTB100, which expresses low levels of Ep-CAM, and the Ep-CAM-negative HT1080 fibrosarcoma cell line was assessed. The results obtained in colorimetric MTT cell viability assays after 72 h Incubation in the presence of wild type immunotoxin (wt) or IMMPA are shown in Figure 10. IMMPA was equally potent as the wt immunotoxin on MCF-7 cells, but showed an about 1 log loss of cytotoxic activity against normal breast control cells, which are supposed not to express MMPs. This finding is indicative for a gain in tumor-specficity provided by the MMP-cleavable site in IMMPA, which replaces the furin site of the wt immunotoxin. Neither of the immunotoxins showed measurable cytotoxicity against the Ep-CAM-negative fibrosarcoma cell line.

**TABLE 1**

| Cell Line | Tissue | EpCAM expression | MMPs activity |
|---|---|---|---|
| MCF-7 | breast carcinoma | +++ | (+++) |
| MDA-MB-231 | breast carcinoma | + | (++) |
| RC-6 | norm. breast tissue (imm.) | +++ | ? |
| HTB-100 | norm. breast tissue (imm.) | - | ? |
| HTB-100/EpCAM | norm. breast tissue (imm.) | +/++ | ? |
| HT1080 | fibrosarcoma | - | (+++) |
| SW2 | small cell lung carcinoma | +++ | ? |
| BEAS-2B | norm. hu bronch. ep. (imm.) | +++ | ? |
| HT29 | colon carcinoma | +++ | ? |
| Colo320 | colon carcinoma | - | ? |
| Vero | norm. simian kidney | - | - |
| Vero/EpCAM | norm. simian kidney | + | - |
| COS-7 | norm. simian kidney | - | - |

| | | | |
|---|---|---|---|
| imm = SV40-immotalized normal human cell line | | | |

### REFERENCES

1. Oppenheimer NJ, Bodley JW (1981) Diphtheria toxin. Site and configuration of ADP-ribosylation of diphthamide in elongation factor 2. J Biol Chem JID - 2985121 R 256:8579-8581
2. Kreitman RJ (1999) Immunotoxins in cancer therapy. Curr Opin Immunol 11:570-578
3. Kreitman RJ (2000) Immunotoxins. Expert Opin Pharmacother 1:1117-1129
4. Grossbard ML, Nadler LM (1993) Monoclonal antibody therapy for indolent lymphomas. Semin Oncol 20:118-135
5. Wahl RL (1994) Experimental radioimmunotherapy. A brief overview. Cancer 73:989-992
6. Grossbard ML, Fidias P (1995) Prospects for immunotoxin therapy of non-Hodgkin's lymphoma. Clin Immunol Immunopathol 76:107-114
7. Jurcic JG, Caron PC, Scheinberg DA (1995) Monoclonal antibody therapy of leukemia and lymphoma. Adv Pharmacol 33:287-314
8. Lewis JP, DeNardo GL, DeNardo SJ (1995) Radioimmunotherapy of lymphoma: a UC Davis experience. Hybridoma 14:115-120
9. Uckun FM, Reaman GH (1995) Immunotoxins for treatment of leukemia and lymphoma. Leuk Lymphoma 18:195-201
10. Kreitman RJ, Wilson WH, Bergeron K, Raggio M, Stetler-Stevenson M, FitzGerald DJ, Pastan I (2001) Efficacy of the anti-CD22 recombinant immunotoxin BL22 in chemotherapy-resistant hairy-cell leukemia. N Engl J Med 345:241-247
11. Schwartzberg LS (2001) Clinical experience with edrecolomab: a monoclonal antibody therapy for colorectal carcinoma. Crit Rev Oncol Hematol JID - 8916049 40:17-24
12.Adkins JC, Spencer CM (1998) Edrecolomab (monoclonal antibody 17-1 A). Drugs JID - 7600076 56:619-626
13. Litvinov SV, Velders MP, Bakker HA, Fleuren GJ, Warnaar SO (1994) Ep-CAM: a human epithelial antigen is a homophilic cell-cell adhesion molecule. J Cell Biol JID - 0375356 125:437-446
14. Willuda J, Honegger A, Waibel R, Schubiger PA, Stahel R, Zangemeister-Wittke U, Pluckthun A (1999) High thermal stability is essential for tumor targeting of antibody fragments: engineering of a humanized anti-epithelial glycoprotein-2 (epithelial cell adhesion molecule) single-chain Fv fragment. Cancer Res JID - 2984705R 59:5758-5767
15.Proca DM, Niemann TH, Porcell Al, DeYoung BR (2000) MOC31 immunoreactivity in primary and metastatic carcinoma of the liver. Report of findings and review of other utilized markers. Appl Immunohistochem Mol Morphol JID - 100888796 8:120-125
16. Pavlovskis OR, Gordon FB (1972) Pseudomonas aeruginosa exotoxin: effect on cell cultures. J Infect Dis JID - 0413675 125:631-636
17. Leppla SH (1976) Large-scale purification and characterization of the exotoxin of Pseudomonas aeruginosa. Infect Immun JID - 0246127 14:1077-1086
18. Saleh MN, Posey JA, Khazaeli MB, Thurmond LM, Khor SP, Lampkin TA, Wissel PS, LoBuglio AF (1998) Phase I trial testing multiple doses of humanized monoclonal antibody (MAb) 3622W94. ASCO 1998 meeting #1680 (Abstract)
19.Raum T, Gruber R, Riethmuller G, Kufer P (2001) Anti-self antibodies selected from a human IgD heavy chain repertoire: a novel approach to generate therapeutic human antibodies against tumor-associated differentiation antigens. Cancer Immunol Immunother JID - 8605732 50:141-150
20. Kroesen BJ, Nieken J, Sleijfer DT, Molema G, de Vries EG, Groen HJ, Helfrich W, The TH, Mulder NH, de Leij L (1997) Approaches to lung cancer treatment using the CD3 x EGP-2-directed bispecific monoclonal antibody BIS-1. Cancer Immunol Immunother JID - 8605732 45:203-206
21. Haller DG (2001) Update of clinical trials with edrecolomab: a monoclonal antibody therapy for colorectal cancer. Semin Oncol 28:25-30
22. Riethmuller G, Holz E, Schlimok G, Schmiegel W, Raab R, Hoffken K, Gruber R, Funke I, Pichlmaier H, Hirche H, Buggisch P, Witte J, Pichlmayr R (1998) Monoclonal antibody therapy for resected Dukes' C colorectal cancer: seven-year outcome of a multicenter randomized trial. J Clin Oncol JID - 8309333 16:1788-1794
23.Dencausse Y, Hartung G, Franz A, Strum J, Edler L, Bornbusch D, Gonnermann M, Post S, Hehlmann R, Queisser W (2000) Prospective randomized study of adjuvant therapy with edrecolomab (PANOREX) of stage II colon cancer: Interum analysis. Ann.Oncol. 11:47(Abstract)
24.Sizmann N and Korting HC. Prolonged Urticaria with 17-1A Antibody. BMJ 317:1631.
25. Naundorf S, Preithner S, Mayer P, Lippold S, Wolf A, Hanakam F, Fichtner I, Kufer P, Raum T, Riethmuller G, Baeuerle PA, Dreier T. In vitro and in vivo activity of MT201, a fully human monoclonal antibody for pancarcinoma treatment. Int J Cancer 100(1):101-10, 2002.
26. Willuda J, Honegger A, Waibel R, Schubiger PA, Stahel R, Zangemeister-Wittke U, Pluckthun A. High thermal stability is essential for tumor targeting of antibody fragments: engineering of a humanized anti-epithelial glycoprotein-2 (epithelial cell adhesion molecule) single-chain Fv fragment. Cancer Res 59(22):5758-67, 1999

## Claims

1. An immunotoxin comprising
(a) an antibody or antibody fragment that binds to Ep-CAM on the surface of a cancer cell; and
(b) a modified toxin comprising a Pseudomonas exotoxin A (ETA) moiety having a furin site replaced with a cancer-associated protease site recognized by metalloproteinase-2 (MMP-2), metalloproteinase-9 (MMP-9) or a combination thereof.

2. The immunotoxin of claim 1, wherein the immunotoxin is internalized by the cancer cell.

3. The immunotoxin of claim 1 or 2, wherein the antibody or antibody fragment that binds to Ep-CAM is a humanized antibody or antibody fragment that binds to the extracellular domain of human Ep-CAM and comprises complementarity determining region sequences derived from a MOC-31 antibody.

4. An immunotoxin of any one of claims 1 to 3 comprising VB4-845 having a furin site replaced with an MMP-2 or MMP-9 protease site or a combination thereof.

5. The immunotoxin of any one of claims 1 to 4, wherein the cancer-associated protease site comprises the sequence selected from the group consisting of GPLGMLSQ and GPLGLWAQ.

6. The immunotoxin of any one of claims 1 to 5 for use in treating cancer.

7. Use of an effective amount of an immunotoxin of any one of claims 1 to 5 for the manufacture of a medicament to treat a mammal with cancer.

8. The immunotoxin of claim 6 or the use of claim 7, wherein the cancer is selected from the group consisting of colorectal cancer, breast cancer, ovarian cancer, pancreatic cancer, head and neck cancer, bladder cancer, gastrointestinal cancer, prostate cancer, small cell and non small cell lung cancer, sarcomas, gliomas, T- and B-cell lymphomas.

9. The use of claim 7 or 8, wherein the mammal is human.

10. A pharmaceutical composition comprising an immunotoxin according to any one of claims 1 to 5 and a pharmaceutically acceptable carrier, diluent or excipient.

## Patentansprüche

1. Immuntoxin, umfassend
(a) einen Antikörper oder ein Antikörperfragment, der/das an Ep-CAM auf der Oberfläche einer Krebszelle bindet; und
(b) ein modifiziertes Toxin, das eine Pseudomonas-Exotoxin A (ETA)-Einheit mit einer Furinstelle umfasst, die durch eine Krebs-assoziierten Proteasestelle ersetzt ist, die durch Metalloproteinase-2 (MMP-2), Metalloproteinase-9 (MMP-9) oder eine Kombination davon erkannt wird.

2. Immuntoxin nach Anspruch 1, wobei das Immuntoxin durch die Krebszelle aufgenommen wird.

3. Immuntoxin nach Anspruch 1 oder 2, wobei der Antikörper oder das Antikörperfragment, der/das an Ep-CAM bindet, ein humanisierter/s Antikörper oder Antikörperfragment ist, der/das an die extrazelluläre Domäne des menschlichen Ep-CAM bindet und Sequenzen von komplementaritätsbestimmenden Regionen abgeleitet von einem MOC-31-Antikörper umfasst.

4. Immuntoxin nach einem der Ansprüche 1 bis 3, umfassend VB4-845 mit einer Furinstelle, die durch eine MMP-2- oder MMP-9-Proteasestelle oder eine Kombination davon ersetzt ist.

5. Immuntoxin nach einem der Ansprüche 1 bis 4, wobei die Krebs-assoziierte Proteasestelle die Sequenz umfasst, die ausgewählt ist aus der Gruppe bestehend aus GPLGMLSQ und GPLGLWAQ.

6. Immuntoxin nach einem der Ansprüche 1 bis 5 zur Verwendung in der Behandlung von Krebs.

7. Verwendung einer wirksamen Menge eines Immuntoxins nach einem der Ansprüche 1 bis 5 für die Herstellung eines Medikaments zur Behandlung eines Säugers mit Krebs.

8. Immuntoxin nach Anspruch 6 oder Verwendung nach Anspruch 7, wobei der Krebs ausgewählt ist aus der Gruppe bestehend aus Kolorektalkrebs, Brustkrebs, Ovarialkrebs, Bauchspeicheldrüsenkrebs, Hals- und Kopfkrebs, Blasenkrebs, gastrointestinalem Krebs, Prostatakrebs, kleinzelligem und nichtkleinzelligem Lungenkrebs, Sarkomen, Gliomen, T- und B-Zell-Lymphomen.

9. Verwendung nach Anspruch 7 oder 8, wobei der Säuger ein Mensch ist.

10. Arzneimittel umfassend ein Immuntoxin nach einem der Ansprüche 1 bis 5 und ein(en) pharmazeutisch verträglichen/s Träger, Verdünnungsmittel oder Excipienten.

## Revendications

1. Immunotoxine comprenant :
(a) un anticorps ou un fragment d'anticorps qui se lie à Ep-CAM à la surface d'une cellule cancéreuse; et
(b) une toxine modifiée comprenant un fragment de l'exotoxine A (ETA) de Pseudomonas ayant un site furine remplacé par un site de protéase associée à un cancer reconnu par une métalloprotéinase-2 (MMP-2), une métalloprotéinase-9 (MMP-9) ou une combinaison de celles-ci.

2. Immunotoxine selon la revendication 1, ladite immunotoxine étant internalisée par la cellule cancéreuse.

3. Immunotoxine selon la revendication 1 ou 2, dans laquelle l'anticorps ou le fragment d'anticorps qui se lie à Ep-CAM est un anticorps humanisé ou un fragment d'anticorps qui se lie au domaine extracellulaire de Ep-CAM humain et comprend des séquences de région déterminant la complémentarité dérivées d'un anticorps MOC-31.

4. Immunotoxine selon l'une quelconque des revendications 1 à 3 comprenant VB4-845 ayant un site furine remplacé par un site de protéase MMP-2 ou MMP-9 ou une combinaison de celles-ci.

5. Immunotoxine selon l'une quelconque des revendications 1 à 4, dans laquelle le site de protéase associée à un cancer comprend la séquence choisie parmi le groupe constitué par GPLGMLSQ et GPLGLWAQ.

6. Immunotoxine selon l'une quelconque des revendications 1 à 5 l'usage dans le traitement de cancer.

7. Utilisation d'une quantité efficace d'une immunotoxine selon l'une quelconque des revendications 1 à 5 pour la fabrication d'un médicament pour traiter un mammifère avec un cancer.

8. Immunotoxine selon la revendication 6 ou l'utilisation selon la revendication 7, dans laquelle le cancer est choisi parmi le groupe constitué par le cancer colorectal, le cancer du sein, le cancer de l'ovaire, le cancer du pancréas, le cancer de la tête et du cou, le cancer de la vessie, le cancer gastro-intestinal, le cancer de la prostate, le cancer du poumon à petites cellules et le cancer du poumon non à petites cellules, les sarcomes, les gliomes, les lymphomes à cellules T et les lymphomes à cellules B.

9. Utilisation selon la revendication 7 ou 8, dans laquelle le mammifère est un humain.

10. Composition pharmaceutique comprenant une immunotoxine selon l'une quelconque des revendications 1 à 5 et un support pharmaceutiquement acceptable, diluant ou excipient.
